# EUROPEAN PATENT APPLICATION

(11) **EP 4 360 650 A1**
(43) Date of publication of application: **01.05.2024**
(21) Application number: 22828537.5
(22) Date of filing: 24.06.2022
(51) Int. Cl.: A61K 45/00, A61K 31/192, A61K 31/195, A61K 31/40, A61K 31/42, A61K 31/4439, A61K 31/7088, A61P 35/00, C12Q 1/02, C12Q 1/68, G01N 33/53

(54) **NOVEL THERAPEUTIC AGENT THAT SUPPRESSES METASTASIS AND PROLIFERATION OF OSTEOSARCOMA AND GLIOMA**

(30) Priority: 25.06.2021 JP 2021106120
(71) Applicant: Japanese Foundation For Cancer Research, Tokyo 135-8550 (JP)
(72) Inventor: TAKAGI Satoshi, Tokyo 135-8550 (JP); KATAYAMA Ryohei, Tokyo 135-8550 (JP)
(74) Representative: Prüfer & Partner mbB Patentanwälte · Rechtsanwälte
(86) International application number: PCT/JP2022/025309
(87) International publication number: WO 2022/270621

(57) **Abstract**

It has been revealed that lysophosphatidic acid (LPA) functions as a mediator that augments invasive ability in osteosarcoma. Furthermore, it has been found that LPAR1, which is a receptor for LPA, is highly expressed in osteosarcoma and glioma, and the LPA-LPAR1 interaction is involved in the metastasis and proliferation of osteosarcoma and glioma. Examinations carried out on the basis of these findings have found that LPAR1 antagonists can serve as therapeutic agents for suppressing the metastasis and proliferation of osteosarcoma and glioma.

## Description

### Technical Field

The present invention relates to a novel therapeutic agent that suppresses the metastasis and proliferation of osteosarcoma and glioma. Specifically, the present invention relates to a therapeutic agent for osteosarcoma and glioma by targeting LPAR1.

### Background Art

Osteosarcoma, a malignant tumor that forms in bones, is a tumor that is heterogeneous in terms both of histology and genetics. Although osteosarcoma is the most frequent tumor among malignant tumors that directly develop in bones, the incidence is as low as 1 to 3 in 1,000,000 individuals, and thus osteosarcoma is a rare cancer. In spite of the peak age of onset being in the childhood and in adolescents and young adults (AYAs), large amounts of chemotherapeutics are administered before and after surgery. This generates concerns about the influence on development and fertility issues common in AYAs, and later onset of cancer in the adulthood. For osteosarcoma, it is said that mutations in TP53 gene and RB1 gene initially causes chromosomal instability and subsequently causes other oncogenic mutations, thereby leading to the onset of polyclonal tumor with metastasis (Non Patent Literatures 1, 2). Accordingly, the very heterogenous nature of the tumor makes it difficult to treat.

Because osteosarcoma is not only a rare cancer but also a very heterogenous tumor as described above, no improvement has been made in survival rates for metastatic osteosarcoma over the past 40 years. The most frequent metastasis is to the lung, 10 to 20% of the patients have pulmonary nodules at initial diagnosis, and 80% or more of the patients have undergone metastasis to the lung at recurrence (Non Patent Literatures 3, 4).

While the survival rate of osteosarcoma patients has been gradually improved since chemotherapeutics were introduced and treatment using chemotherapeutics in combination with surgical operations emerged in the 1970s, the survival rate of osteosarcoma patients with metastasis is still low, there is no effective therapeutic method for osteosarcoma patients with lung metastasis, and the survival rate is said to be 19 to 37% (Non Patent Literatures 3, 5 to 8). Therefore, suppressing metastasis in treatment of osteosarcoma is an important task for improved prognosis.

While high doses of methotrexate, doxorubicin, cisplatin, and ifosfamide have been introduced to the primary treatment of chemotherapy, there is no consensus with respect to optimum combination therapy, therapeutic methods for metastatic or recurrent osteosarcoma and the like. If a therapeutic agent targeting a molecule specifically expressed in osteosarcoma is successfully obtained, it becomes possible to provide a novel therapeutic agent and therapeutic method. Also for establishing an optimum therapeutic method, elucidation of a molecule involved in the proliferation and metastasis of osteosarcoma is of significance.

On the other hand, glioma is a malignant brain tumor that develops from neuroglial cells, and glioma with the highest malignancy is called glioblastoma. In addition to headache, various symptoms including limb paralysis, impaired vision and visual acuity are presented depending on the site where tumor has developed or grown. If tumor cells of glioma have invaded into normal brain tissue, it is difficult to completely remove those cells, and thus glioma is regarded as one of tumors with poor prognosis. For glioblastoma, which has the highest malignancy among gliomas, causative gene mutations including IDH and p53 gene mutations have been reported, but molecular target linked to treatment has not been reported yet.

Therapeutic methods for glioma involve surgical treatment to remove tumor as much as possible while the motor functions, language functions, and the like are preserved. However, total excision of tumor is difficult as described above, and recurrence prevention is to be attempted through radiotherapy and chemotherapy. Treatment with administration of temozolomide or bevacizumab is performed in combination with radiotherapy; however, even such treatment quite frequently results in undesirable recurrence. Accordingly, development of a more effective therapeutic agent, in particular, a molecular target drug, is desired.

### Citation List

### Non Patent Literature

Non Patent Literature 1: Bousquet M, et al., 2016, Ann. Oncol. Vol. 27, pp. 738-744.
Non Patent Literature 2: Ribi S, et al., 2015, Oncotarget, Vol. 6, pp. 7727-7740.
Non Patent Literature 3: Kager L, et al., 2003, J. Clin. Oncol. Vol. 21, pp. 2011-2018.
Non Patent Literature 4: Meyers PA, et al., 1993, J. Clin. Oncol. Vol. 11, pp. 449-453.
Non Patent Literature 5: Aljubran AH et al., 2009, Ann. Oncol. Vol. 20, pp. 1136-1141.
Non Patent Literature 6: Gok Durnali A, et al., 2016, PLoS One 2016; 11: e0152621.
Non Patent Literature 7: Salah S, et al., 2014, Mol. Clin. Oncol. Vol. 2, pp. 811-816.
Non Patent Literature 8: Briccoli A, 2010, Surg. Oncol. Vol. 19, pp. 193-199.
Non Patent Literature 9: O'Donnell VB et al., 2014, Circ. Res. Vol. 114, pp. 1185-1203.
Non Patent Literature 10: Chatterjee M., 2020, J. Thromb. Haemost. Vol. 18, pp. 543-557.

### Summary of Invention

### Technical Problem

An object of the present invention is to provide an effective therapeutic agent and treatment method for osteosarcoma and glioma, in particular, a therapeutic agent that suppresses the metastasis and proliferation of tumor. Osteosarcoma involves lung metastasis with relatively high probability, no effective therapeutic method is available for osteosarcoma patients with lung metastasis, and most of the causes of patient death are respiratory failure due to lung metastasis; therefore, if metastases including lung metastasis were successfully suppressed, prognosis could be improved. For glioma, which is difficult to completely remove glioma by surgical treatment, the development of a therapeutic agent that suppresses tumor proliferation is expected to be promising. Since no effective molecular target drug has been developed for osteosarcoma and glioma, finding a molecule specifically expressed in those tumors and suppressing its functions should lead to the development of a novel therapeutic agent.

As shown in detail later, the present inventors have found that LPAR1 is highly expressed in osteosarcoma and completed the present invention based on this finding. In addition, high LPAR1 expression was also found in glioma, and effects of LPAR1 antagonists were then analyzed. It will become possible to provide an effective therapeutic agent for osteosarcoma and glioma, for which no effective therapeutic agent has been previously available.

### Solution to Problem

The present invention relates to the following pharmaceutical composition, prognostication assistance method, and drug selection method.
(1) A pharmaceutical composition for treating osteosarcoma or glioma, the pharmaceutical composition containing a compound that suppresses LPAR1 expression as an active ingredient.
(2) The pharmaceutical composition for treating osteosarcoma or glioma according to (1), wherein the compound that suppresses LPAR1 expression is an LPAR1 antagonist or a nucleic acid.
(3) The pharmaceutical composition for treating osteosarcoma or glioma according to (2), wherein the LPAR1 antagonist is a low-molecular-weight compound.
(4) The pharmaceutical composition for treating osteosarcoma or glioma according to (3), wherein the LPAR1 antagonist is any of ONO-7300243, BMS-986020, Ki16425, ONO-3080573, ONO-9780307, ONO-9910539, Ki16198, AM095, AM966, SAR100842, BMS-986278, and analogs thereof.
(5) A method of assisting prognosis for an osteosarcoma patient, wherein LPAR1 expression in disease tissue obtained from the patient is analyzed, and if the LPAR1 expression is high, the risk of metastasis is predicted to be high.
(6) A drug selection method, wherein LPAR1 expression in disease tissue is analyzed, and if LPAR1 expression is detected, the disease is determined to be a subject for LPAR1 antagonist administered.
(7) A screening method for a pharmaceutical composition for treating osteosarcoma or glioma, the screening method including the steps of: bringing a candidate substance into contact with a cell with appreciable LPAR1 expression; and measuring the change of LPAR1 expression.
(8) A treatment method for an osteosarcoma patient or a glioma patient, wherein a specimen is obtained from an osteosarcoma patient or a glioma patient to analyze LPAR1 expression, and if LPAR1 expression is found, the patient is determined to be a subject for administering a compound that suppresses LPAR1 expression and then the compound that suppresses LPAR1 expression is administered.

### Brief Description of Drawings

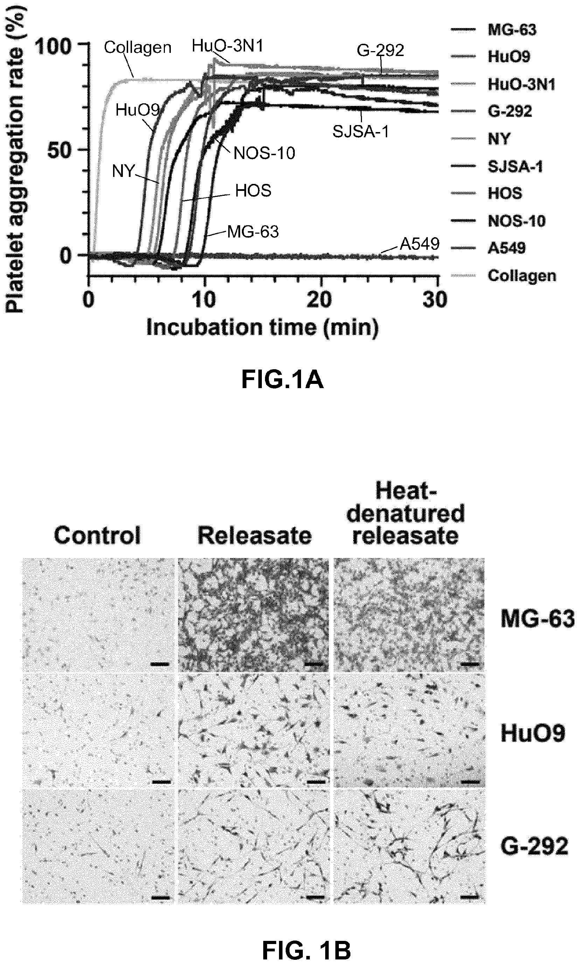

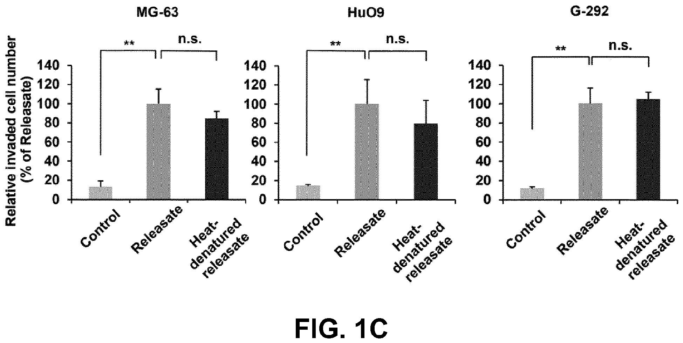

[Figure 1] Figure 1 demonstrates that osteosarcoma cells have high platelet aggregation ability, and the invasive ability is enhanced by a platelet releasate which is released from platelets. Figure 1A is a graph representing the platelet aggregation ability of different types of osteosarcoma cells. Figure 1B and Figure 1C show that platelet releasate released from platelets enhances the invasive ability of osteosarcoma cells. Figure 1B is a series of photomicrographs showing invaded cells, and Figure 1C is a series of graphs with each representing the number of invaded cells.

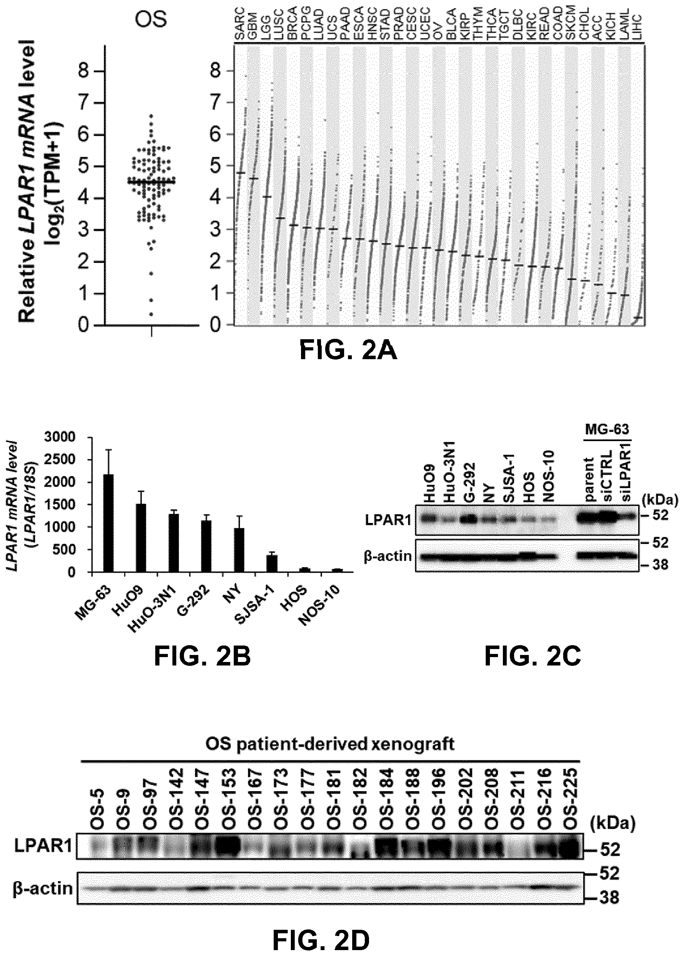

[Figure 2] Figure 2 demonstrates the enhanced expression of LPAR1 in osteosarcoma. Figure 2A is a diagram of analysis of LPAR1 mRNA expression levels with use of RNA sequence data from The Cancer Genome Atlas (TCGA) and TARGET. Figure 2B is a graph representing results of qPCR analysis of LPAR1 mRNA expression in osteosarcoma cell strains, and Figure 2C is a diagram showing results of Western blotting analysis of LPAR1 protein expression in osteosarcoma cell strains. Figure 2D is a diagram showing LPAR1 protein expression in a xenograft derived from an osteosarcoma patient.

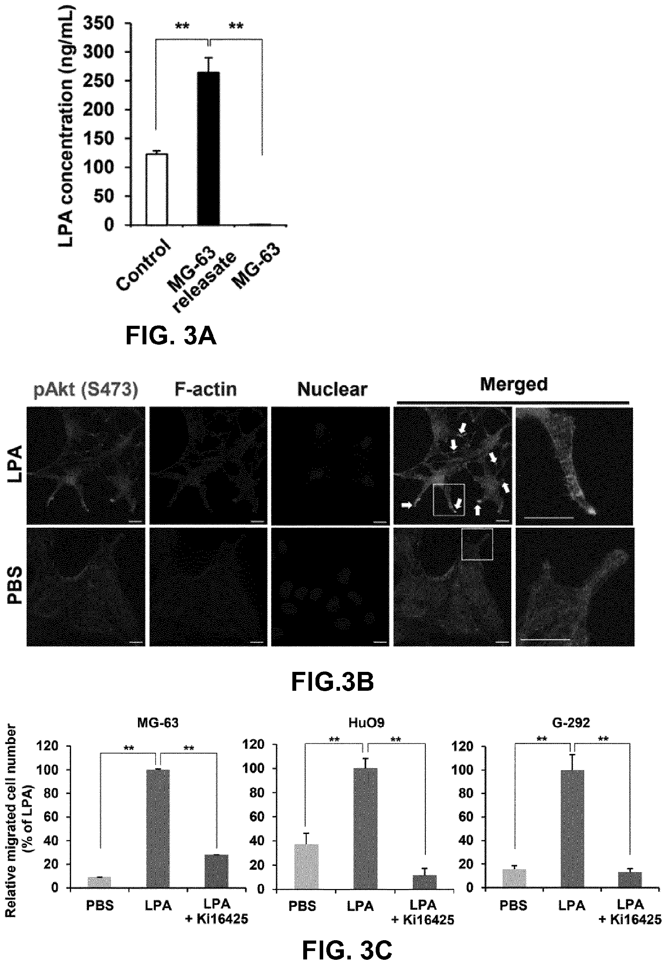

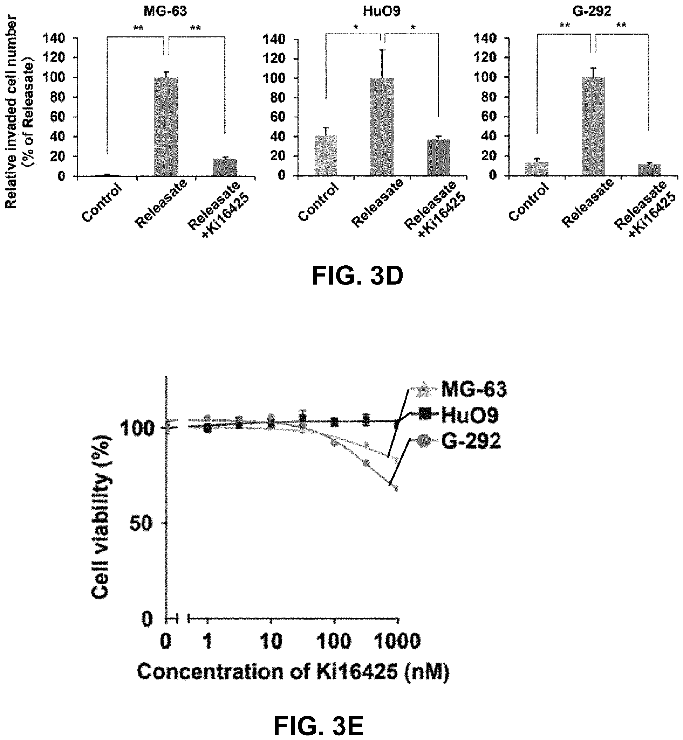

[Figure 3] Figure 3 demonstrates the importance of LPA released from activated platelets for the migration ability and invasive ability of osteosarcoma cells. Figure 3A is a graph obtained through ELISA analysis to confirm the release of LPA from platelets by adding MG-63 osteosarcoma cells to platelet suspension. Figure 3B is a series of fluorescence photomicrographs for analyzing the localization of phosphorylated AKT and F-actin in MG-63 osteosarcoma cells stimulated with LPA. Figure 3C is a series of graphs demonstrating effects of LPA on cell migration ability and the cancellation of the effects of LPA by the LPAR antagonist Ki16425 in different types of osteosarcoma cells. Figure 3D is a series of graphs demonstrating effects of platelet releasate on invasive ability and the cancellation of the effects of platelet releasate by Ki16425 in different types of osteosarcoma cells. Figure 3E is a graph for analyzing the influence of Ki16425 on the proliferation of osteosarcoma cells.

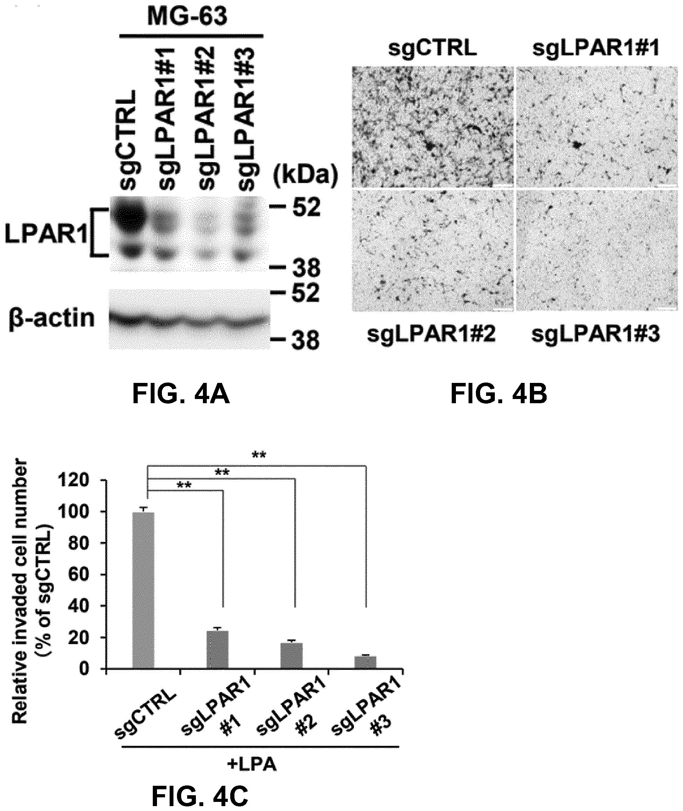

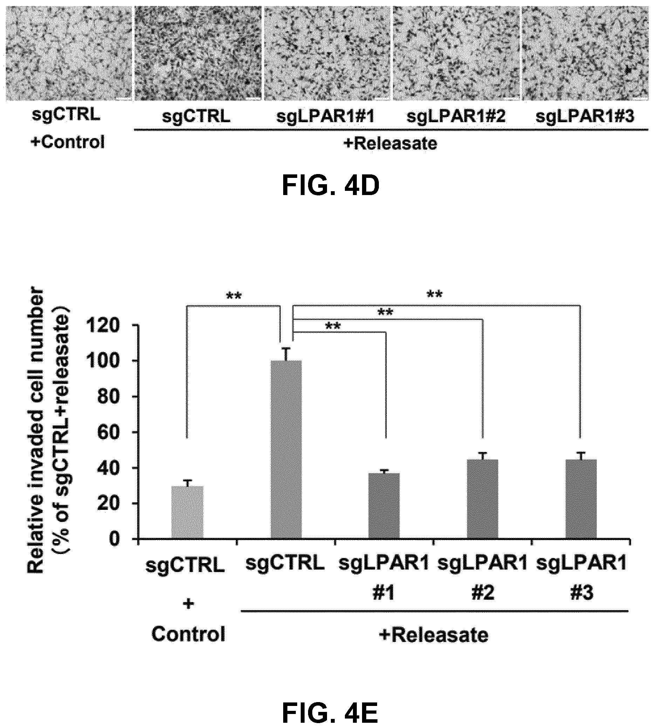

[Figure 4] Figure 4 demonstrates the importance of LPA-LPAR1 interaction for invasion of osteosarcoma cells. Figure 4A is a diagram demonstrating that LPAR1-knockout cells were established by using MG-63 osteosarcoma cells. Figures 4B and 4C demonstrate effects of LPA on invasive ability in LPAR1-knockout cells. Figures 4D and 4E demonstrate effects of platelet releasate on invasive ability in LPAR1-knockout cells.

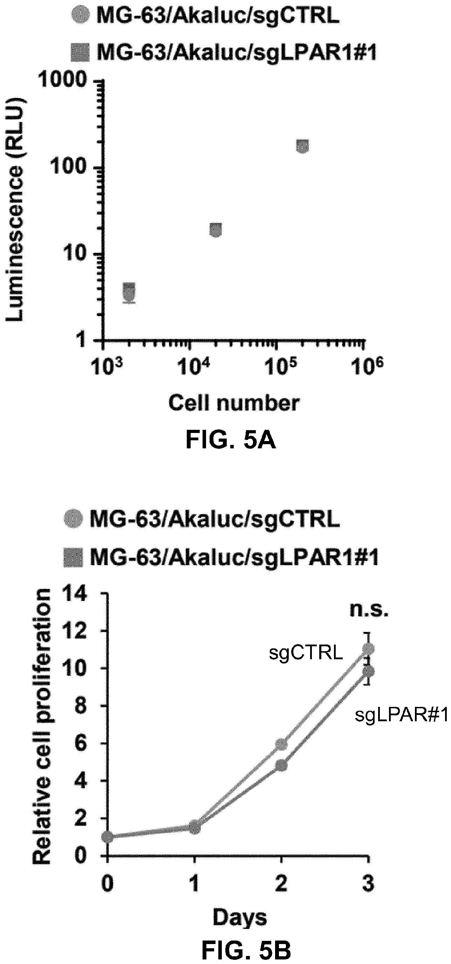

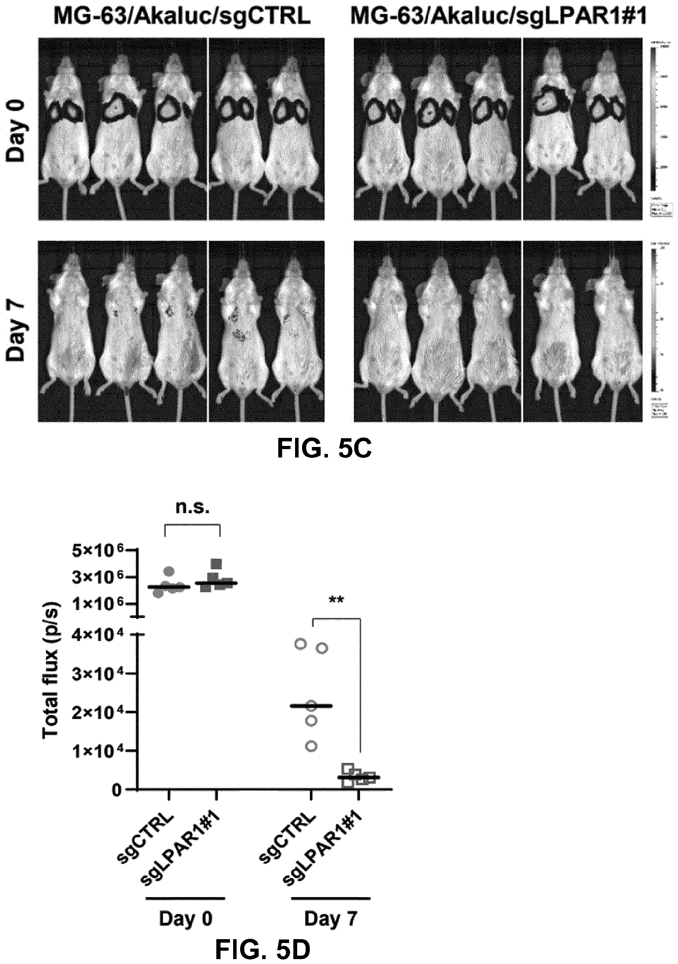

[Figure 5] Figure 5 demonstrates that LPAR1 has an important role in the lung metastasis of osteosarcoma. Figure 5A is a graph showing the luminescence of MG-63 osteosarcoma cells (MG-63/Akaluc/sgCTRL) and LPAR1-knockout MG-63 osteosarcoma cells (MG-63/Akaluc/sgLPAR#1) with Akaluc luciferase introduced therein, and Figure 5B is a graph showing their cell proliferative capacities. Figures 5C and 5D show results of *in vivo* imaging analysis in which MG-63/Akaluc/sgCTRL and MG-63/Akaluc/sgLPAR#1 cells were intravenously injected and AkaLumine was administered 0 days (the day of injection) and 7 days later.

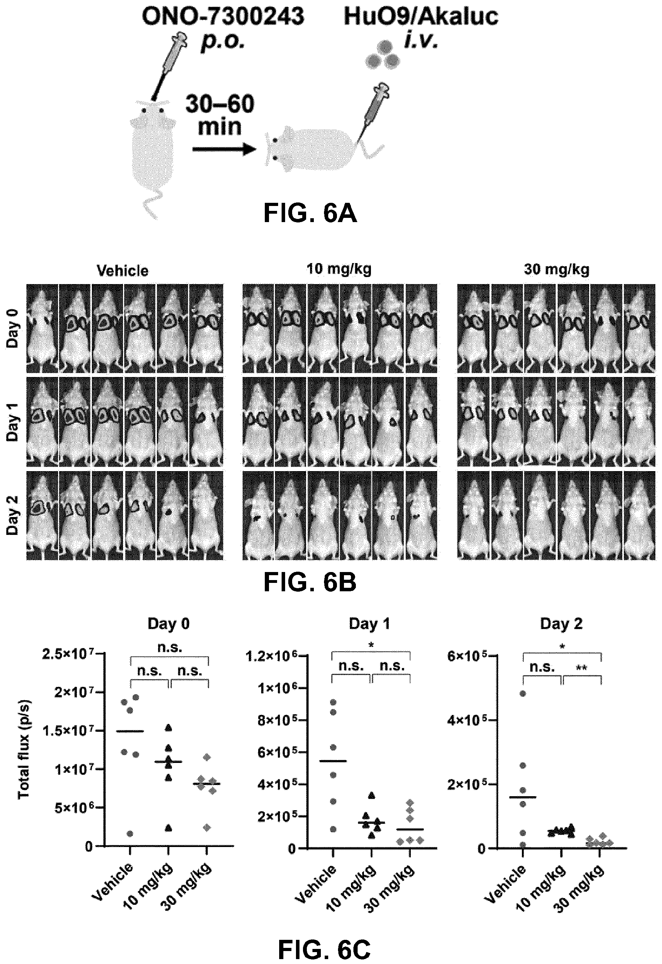

[Figure 6] Figure 6 demonstrates that the lung metastasis of osteosarcoma is suppressed by an LPAR1 antagonist. Figure 6A is a diagram illustrating the procedure of experiment. Figures 6B and 6C show results of *in vivo* imaging analysis demonstrating that the lung metastasis is suppressed by administering a LPAR1 antagonist ONO-7300243 and injecting HuO9/Akaluc osteosarcoma cells.

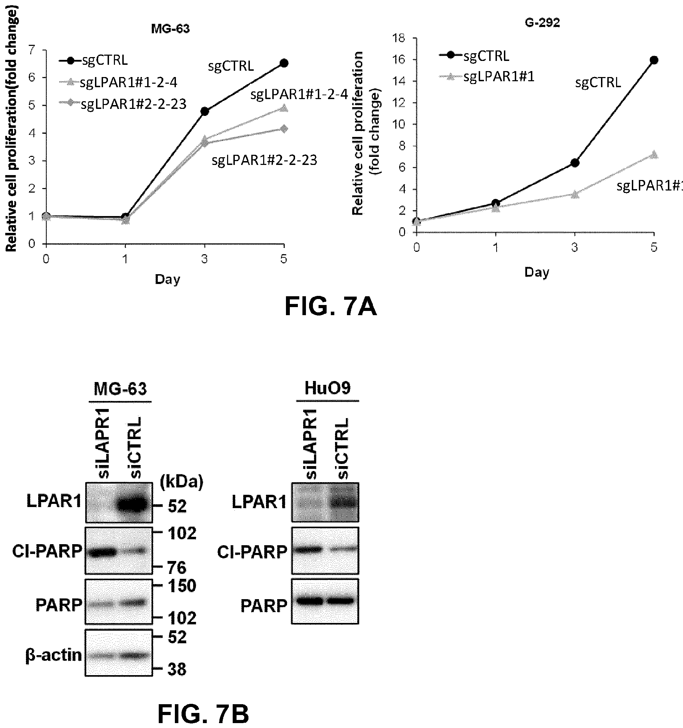

[Figure 7] Figure 7 demonstrates that LPAR1 is involved in the proliferation of osteosarcoma cells. Figure 7A is a series of graphs demonstrating that cell proliferation is suppressed in clone strains obtained by knockout of LPAR1 in the osteosarcoma cells MG-63 and G-292 clone A141B1 (G-292) cells. Figure 7B is a series of diagrams demonstrating that apoptosis is induced through knockdown of LPAR1 by siRNA in the osteosarcoma cells MG-63 and HuO9.

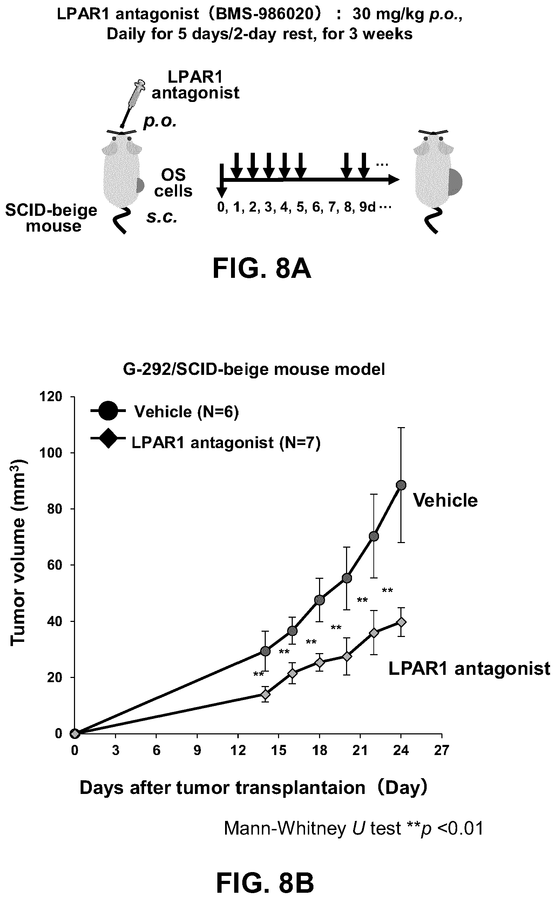

[Figure 8] Figure 8 demonstrates that administration of an LPAR1 antagonist exerts antitumor effect in an osteosarcoma xenograft model. Figure 8A shows the schedule for administration of LPAR1, and Figure 8B shows the change in tumor volume over time.

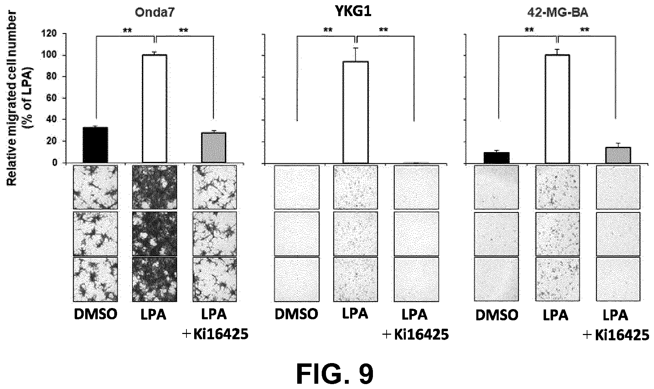

[Figure 9] Figure 9 demonstrates that the mobility of glioblastoma cells is renewed in the presence of LPA.

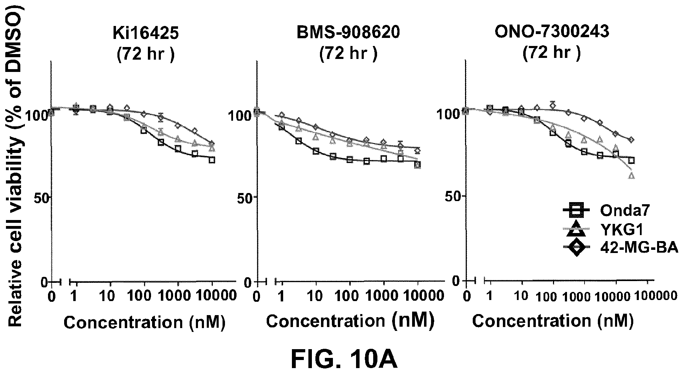

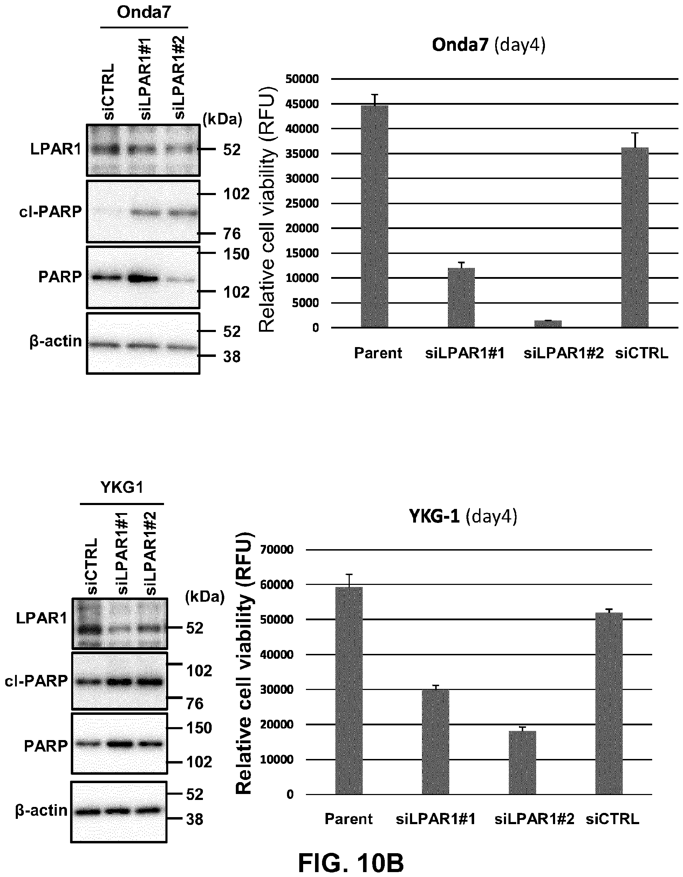

[Figure 10] Figure 10 shows the influence of LPAR1 on cell viabilities. Figure 10A is a series of graphs demonstrating that an LPAR1 antagonist reduces the viability of glioblastoma cells. Figure 10B demonstrates that LPAR1 knockdown results in the reduced viability of glioblastoma cells.

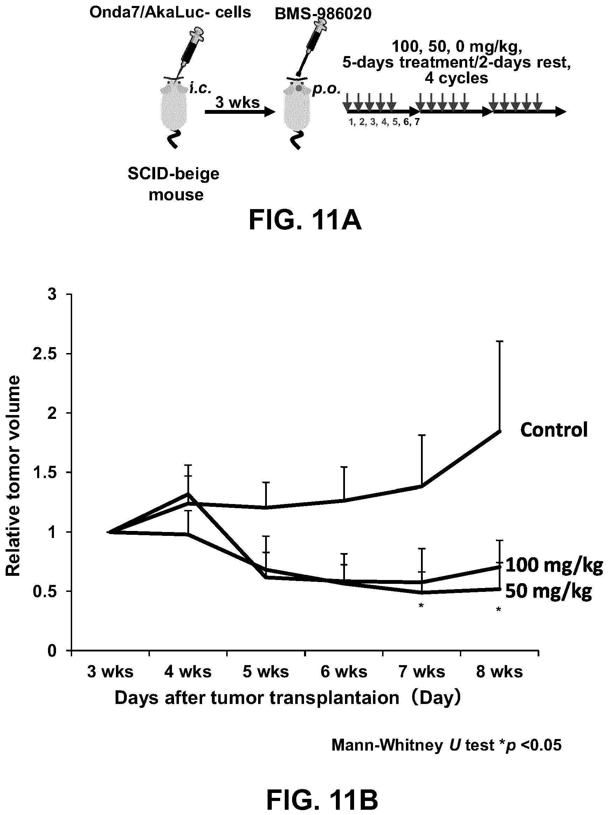

[Figure 11] Figure 11 demonstrates that administration of an LPAR1 antagonist exerts antitumor effect in a glioblastoma xenograft model. Figure 11A shows the schedule of administration of an LPAR1 antagonist, and Figure 11B shows the change in relative tumor volume over time.

### Description of Embodiments

The present inventors have found that osteosarcoma cells have high platelet aggregation ability, and that platelet releasate released from activated platelets augments the invasive ability of osteosarcoma cells. Further analysis revealed that lysophosphatidic acid (LPA) released from activated platelets functions as a mediator to augment invasive ability of osteosarcoma. In addition, finding that expression of LPAR1, which is a receptor for LPA, was dramatically enhanced in osteosarcoma cells and a patient-derived xenograft, the present inventors has reached the concluded that the LPA-LPAR1 interaction is involved in the distant metastasis of osteosarcoma.

Moreover, using a mouse model, the inventors have found that the lung metastasis is suppressed by administering an LPAR1 antagonist, specifically, ONO-7300243, and that the tumor proliferation is suppressed with BMS986020. From the results that cells in which the expression of LPAR1 had been knocked out or knocked down exhibited suppressed proliferation and apoptosis induction, it is found that the proliferation of osteosarcoma can be suppressed by suppressing the expression of LPAR1. Furthermore, as LPAR1 was also highly expressed in glioma, effects of an LPAR1 antagonist was analyzed to find that the LPAR1 antagonist exerts antitumor effect.

Thus, the LPA-LPAR1 interaction is involved in the metastasis and proliferation of osteosarcoma and glioma as described above, and therefore any LPAR1 antagonist that suppresses the metastasis and proliferation may be used. While low-molecular-weight compounds are used herein, such compounds include not only ONO-7300243, BMS-986020, and Ki16425 used herein, but also ONO-3080573, ONO-9780307, ONO-9910539, Ki16198, AM095, AM966, SAR100842, BMS-986278, and analogs thereof. In addition, antibodies and polypeptides that bind to LPAR1 and inhibit its functions can be used. Moreover, the LPAR1 expression itself may be suppressed with a nucleic acid, such as siRNA, antisense RNA, shRNA, and miRNA.

In addition to the existing LPAR1 antagonists, compounds that suppress LPAR1 expression may be obtained by screening for use. Screening for compounds that suppress LPAR1 expression may be performed by adding a candidate substance to the culture broth of LPAR1-expressing cells, specifically osteosarcoma cells or glioma cells, and screening the substance in light of reduction in LPAR1 expression as an indicator.

Moreover, because LPAR1 expression is deeply involved in bone metastasis for osteosarcoma patients, measurement of LPAR1 expression in tumor tissue allows determination of the risk of distant metastasis, i.e., prognosis. Specifically, LPAR1 expression is detected from osteosarcoma tissue obtained by surgery or biopsy, and if the LPAR1 expression is high, it can be determined that the probability of distant metastasis is high. If the probability of distant metastasis is high, distant metastasis can be prevented through a preventive measure such as administering an LPAR1 antagonist. Not only in osteosarcoma and glioma but also in other types of cancer, LPAR1 inhibitors are expected to be effective for high LPAR1 expression, and hence patients for whom an LPAR1 inhibitor should be selected as a therapeutic agent can be determined by examining LPAR1 expression in specimens from patients.

Hereinafter, description will be given with reference to data. Interaction between platelets and cancer as well as release of a bioactive molecule from activated platelets have been reported to be important in the hematogenous metastasis of epithelial tumor, whereas whether they are important in sarcoma is still unclear. Hence, platelet aggregation assay was carried out with eight osteosarcoma cell strains to clarify whether osteosarcoma cells interact with and activate platelets (Figure 1A). Human osteosarcoma cells were obtained from ATCC, Riken BioResource Research Center, or JCRB Cell Bank, and cultured according to a method recommended by the provider.

Platelets were isolated by a conventional method from blood obtained from healthy individuals who had taken no antiplatelet agent for at least 10 days before blood collection. The isolated platelets were suspended in modified Tyrode buffer (137 mM NaCl, 11.9 mM NaHCOs, 0.4 mM Na₂HPO₄, 2.7 mM KCl, 1.1 mM MgCl₂, 5.6 mM glucose) of 2 × 10⁸/mL, 1.2 mM CaCl₂ was added thereto, and the resultant was used for the platelet aggregation assay. Cell suspension (10 µL of 5 × 10⁶ cells/mL cell suspension) or PBS was added to 200 µL of the platelet suspension, and the resultant was analyzed at 37°C for 30 to 60 minutes. Platelet aggregation was measured with a platelet aggregometer (MCM HEMA Tracer 313M, SSR Engineering, Inc.) (Figure 1A).

All the types of osteosarcoma cells had higher platelet activation ability than the lung adenocarcinoma cells A549, which were used as a negative control. Collagen (10 µg/mL, 10 µL was added) was a positive control. That result revealed that all the types of osteosarcoma cells used in the analysis had high platelet aggregability, i.e., platelet activation potential.

The possibility that a bioactive molecule released from activated platelets affects the invasive ability of osteosarcoma cells was examined. The reaction solutions subjected to the platelet aggregation assay were collected and centrifuged with addition of 0.5 µM prostaglandin I₂, and the centrifugal supernatant was collected. The centrifugal supernatant was used as platelet releasate containing a bioactive molecule released from activated platelets.

The osteosarcoma cells MG-63, HuO9, and G-292 were each seeded in an insert (upper chamber) of a Matrigel Invasion Chamber (Corning Incorporated) at 1.5 × 10⁵ cells/0.5 mL with the platelet releasate put in the lower chamber, and left to stand at 37°C for 22 to 24 hours, and then cells on the top surface of the insert were completely wiped away, fixation was performed with 4% paraformaldehyde, and cells on the bottom surface of the membrane of the insert, i.e., invaded cells were stained with 1% crystal violet (Figure 1B). The number of invaded cells was counted, and the relative proportions of such cells are shown in Figure 1C. In any type of the osteosarcoma cells, the number of invaded cells was significantly increased when the platelet releasate was added.

It is known that not only peptide but also lipid in platelet releasate functions as a mediator. For analysis to determine whether peptide or lipid mediator is involved in the invasive ability of osteosarcoma cells, heat treatment was performed at 95°C, under which most proteins denature, for 10 minutes, and the invasive ability was analyzed with a Matrigel Invasion Chamber in the same manner (Figures 1B, 1C). The results showed that the effect was not lost even through the heat treatment, and thus the mediator was expected to be lipid.

Reports with mass spectrometry analysis have shown that lipid mediators, such as TxA2, S1P, and LPA, are released from activated platelets (Non Patent Literatures 9, 10). Although data are not shown herein, expression of lipid mediator receptors (TBA2R, S1PR1 to S1PR5, LPAR1 to LPAR6) was examined in different tumors by using RNA sequence data from TCGA and TARGET databases. LPAR1, LPAR6, S1PR1, and S1PR3 were found to be highly expressed in osteosarcoma. Furthermore, comparison among different cancer types confirmed that LPAR1 was most highly expressed in osteosarcoma (OS) and sarcoma (SARC) (Figure 2A). The gene expression data from TCGA and TARGET are those of tumor tissue, and hence include data not only of tumor cells but also of interstitial cells, epithelial cells, immunocytes, and so on. In view of this, although data are not shown herein, analysis was carried out by using the Cancer Cell Line Encyclopedia (CCLE) database, which reflects gene expression in cancer cell strains. Some osteosarcoma cell strains exhibited significantly higher LPAR1 expression than Ewing's sarcoma family of tumors and chondrosarcoma.

Furthermore, qPCR (Figure 2B) and Western blotting (Figure 2C) confirmed that LPAR1 was highly expressed in the following six out of the eight types of osteosarcoma cells: MG-63, HuO9, HuO-3N1, G-292, NY, and SJSA-1. In addition, Western blotting for osteosarcoma-derived xenograft samples established from patients revealed that approximately 80% thereof (15/19) were LPAR1-positive (Figure 2D).

Analysis by ELISA (human lysophosphatidic acid ELISA kit, CUSABIO TECHNOLOGY LLC) was carried out to determine whether LPA is released from platelets through the interaction with osteosarcoma cells. At 37°C, 5 × 10⁴ MG-63 cells with 200 µL of platelet suspension or MG-63 cells alone were left to stand for 30 minutes. Prostaglandin I₂ was added to reach 0.5 µM, the resultant was centrifuged, and LPA was then detected by ELISA. As shown in Figure 3A, the results showed that no LPA was detected in the supernatant for the case with MG-63 cells alone, and by contrast large amounts of LPA were contained for the case with MG-63 cells and platelets mixed together. In addition, significant release of LPA was shown by comparing with centrifugal supernatant of non-activated platelets (control).

LPA receptors are G-protein-coupled receptors, and LPAR1 is known to activate three G proteins, G_{αi/0}, G_{αq/11}, and Gα_{12/13}, and to activate a signal transduction system including a PI3K/AKT pathway. MG-63 cells were cultured with serum-free MEM medium overnight, then treated with 100 nM LPA for 4 hours, immunostained through a conventional method with an anti-phosphorylated AKT antibody (an antibody to detect phosphorylation of S473), rhodamine-labeled phalloidin (for binding to F actin), and Hoechst 33342 (for staining nuclei), and observed with a microscope (Figure 3B). Activated AKT was found in the cells with addition of LPA, and further found to be localized in the same region as F actin as indicated by arrows. Although results are not shown herein, time-lapse analysis with a microscope was carried out to analyze effects of LPA on osteosarcoma cells, and it was observed that osteosarcoma cells formed numerous protrusions (pseudopodia) as a result of the LPA treatment. Not only for MG-63 cells but also for HuO9 cells, the colocalization of phosphorylated AKT and F-actin was observed in protruding parts of cell membranes.

Next, the influence of LPA on migration ability and invasive ability was analyzed (Figure 3C). Analysis of migration ability was carried out by using a Transwell chamber (Corning Incorporated, 8.0 µm pore). In an insert, 1 × 10⁵ MG-63, HuO9, or G-292 cells suspended in 0.3 mL of serum-free MEM medium were seeded with 10 nM LPA as a chemoattractant in 1.25 mL of serum-free MEM medium put in the lower chamber, left to stand for 4 to 6 hours and then fixed, and subjected to analysis of migration ability (LPA). Separately, cells after being pretreated with 100 nM Ki16425 (Santa Cruz Biotechnology, Inc.), which is an LPAR antagonist, for 1 hour were collected, seeded in an insert, and subjected to analysis of migration ability in the presence of 100 nM Ki16425 (LPA+Ki16425). All the types of cells exhibited migration ability largely enhanced by LPA, whereas the cells treated with Ki16425 exhibited largely reduced migration ability even with addition of LPA.

In addition, analysis of invasive ability was carried out by using a Matrigel Invasion Chamber in the same manner as for Figure 1C (Figure 3D). Analysis with addition of the platelet releasate to the underlying chamber revealed that the platelet releasate allowed all the types of cells to acquire invasive ability. The cells treated with Ki16425 in advance exhibited largely reduced invasive ability. As shown in Figure 3E, reduced cell viabilities were found in G-292 and MG-63 cells cultured for 72 hours with addition of Ki16425 at high concentrations of 500 nM or more. Under the conditions in the tests of migration ability and invasive ability, which involved treatment at a relatively low concentration of 100 nM for a relatively short time of 4 to 6 hours (migration ability) or 22 to 24 hours (invasive ability), no cytotoxicity was found. Accordingly, the reduction in migration ability and invasive ability due to Ki16425 was confirmed not to be caused by cytotoxicity to the cells. From the above results, it was demonstrated that the bioactive molecule in the platelet releasate is LPA and the enhancement of the migration ability and invasive ability of osteosarcoma cells occurs through the action of LPA as a mediator.

Ki16425 exhibits inhibitory effect to all of LPAR1, LPAR2, and LPAR3, although the degree of inhibition differs among them. To determine which LPAR functions to the invasive ability of osteosarcoma, LPAR1-knockout cells were established from MG-63 cells by using a CRISPR/CAS9 system. The established cells are referred to as sgLPAR1#1 to sgLPAR1#3. Western blotting confirmed that all of sgLPAR1#1 to sgLPAR1#3 had lost LPAR1 expression (Figure 4A).

Analysis of invasive ability was carried out with those knockout cells. In an insert, 1 × 10⁵ cells of each type were seeded with addition of 10 nM LPA to the underlying chamber of a Matrigel Invasion Chamber, fixed after 22 to 24 hours incubation, and stained with crystal violet, and the number of cells was counted (Figures 4B, 4C). All the types of knockout cells exhibited significantly lower invasive ability than a control.

The same analysis was carried out with the platelet releasate released from activated platelets. The platelet releasate equivalent to 10 nM LPA was added to the underlying chamber, then cultured for 22 to 24 hours, and the resultant was fixed, stained, and analyzed (Figures 4D, 4E). All the types of knockout cells exhibited no enhancement in invasive ability due to the platelet releasate. From the results, it was suggested that the lung metastasis of osteosarcoma is mediated by the LPA-LPAR1 interaction.

Next, functions of LPAR1 in the lung metastasis of osteosarcoma were analyzed *in vivo* by using a mouse model. Akaluc luciferase was introduced into MG-63/sgCTRL and MG-63/sgLPAR#1 cells to obtain the following Akaluc luciferase-expressing cells: MG-63/Akaluc/sgCTRL and MG-63/Akaluc/sgLPAR#1 cells. The cells were seeded at different number of cells shown in Figure 5A and treated with 50 µM AkaLumine-HCL, and luminescence was measured with a Mithras LB940 plate reader (Berthold Technologies GmbH & Co. KG). The cells obtained were confirmed to be emitting light at the intensities corresponding to the number of cells. Cell proliferation was measured with CellTiter-Gro reagent (Promega Corporation). No large difference in proliferation was found between the two types of cells (Figure 5B).

Into each female SCID-beige mouse (C.B-lgh-1b/GbmsTac-Prkdc^{scid}-Lyst^{bg}N7, Charles River Laboratories Japan, Inc.), 1 × 10⁶ MG-63/Akaluc/sgCTRL or MG-63/Akaluc/sgLPAR#1 cells were intravenously injected, 3 hours and 7 days after, 100 µL of 5 mM AkaLumine-HCL was intraperitoneally injected and *in vivo* luminescence imaging (bioluminescent imaging, BLI) was carried out. The BLI was carried out by using an IVIS imaging system (PerkinElmer, Inc.). Figure 5C shows the images, and Figure 5D shows Total Flux measured with the IVIS. On the day of intravenous injection of cells (Day 0), the both types of cells were captured in lungs to the same degree. Seven days thereafter (Day 7), however, MG-63/Akaluc/sgCTRL cells were surviving in lungs, whereas MG-63/Akaluc/sgLPAR#1 cells, which were LPAR1-knockout cells, were not surviving, and significant difference in Total Flux was found, suggesting that LPAR1 plays an important role in the lung metastasis.

Next, effects of an LPAR1 antagonist was examined. Analysis with a mouse model was carried out by using ONO-7300243 (Cayman Chemical Company), which is one of LPAR1 inhibitors. As illustrated in Figure 6A, after ONO-7300243 was orally administered, 1 × 10⁶ HuO9/Akaluc cells were intravenously injected within 30 minutes to 60 minutes, and BLI was carried out in the same manner. In the experiment, ONO-7300243 was used at different doses of 10 mg/kg and 30 mg/kg.

The BLI was carried out for 1.5 to 3 hours, 1 day, and 2 days after the administration (Figure 6B). Immediately after the administration, it was observed that HuO9/Akaluc cells were captured in lungs at any dose. A day or two later, reduction of osteosarcoma cells captured in lungs was observed in the ONO-7300243 administration group, in contrast to the solvent administration group.

Although there was no significant difference in Total Flux measured with the IVIS (Figure 6C) among the groups on the day of injection of cells, significant difference was found between the solvent administration group and the 30 mg/kg administration group on Day 1, and significant difference was further found between the 10 mg/kg administration group and the 30 mg/kg administration group on Day 2; thus, reduction of osteosarcoma cells captured in lungs depending on the dose was found.

As described above, it was demonstrated that LPAR1-knockout or treatment with an LPAR1 antagonist suppresses the metastasis. Next, effects of LPAR1 expression on cell proliferation were analyzed. As demonstrated in Figure 3E, the experiment with an LPAR1 antagonist found no large effect on cell proliferation. In view of this, analysis with a more sensitive system was carried out to determine whether LPAR1 expression has influence on cell proliferation.

LPAR1 in the osteosarcoma cells MG-63 and G-292 was knocked out by using a CRISPR/Cas9 system, and clone strains of the cells were obtained to compare their cell proliferative capacities (Figure 7A). Suppression of cell proliferation was found in all of the LPAR1-knockout cell strains used.

Examination was carried out to determine what mechanism was responsible for the occurrence of the suppression of cell proliferation. LPAR1 in MG-63 and HuO9 cells was knocked down by using siRNA targeting LPAR (Dharmacon). Increased levels of cleaved PARP (Cl-PARP), which is an apoptosis marker, were found as a result of the knockdown of LPAR1, and hence apoptosis is expected to be induced through knockdown of LPAR1. Accordingly, it is understood that the apoptosis of osteosarcoma cells is induced not only by LPAR1 antagonists but also through suppression of LPAR1 expression by siRNA, and antitumor effect is exerted thereby.

From the above results, it is expected that apoptosis is induced by inhibiting LPAR1 with an antagonist, and therefor proliferation suppression occurs in osteosarcoma. Accordingly, examination was carried out to determine whether the proliferation of osteosarcoma cells subcutaneously transplanted into mice is suppressed by administration of an LPAR1 antagonist (Figure 8).

Into each female SCID-beige mouse, 8.2 × 10⁵ cells of the osteosarcoma cells G-292 were subcutaneously transplanted (Day 0), and on the day after the transplantation (Day 1) oral administration according to a dosing schedule of continuous administration of the LPAR1 antagonist BMS-986020 of 30 mg/kg for 5 consecutive days and withdrawal for 2 days was initiated (Figure 8A). The proliferation of tumor was analyzed by measuring the major axis and minor axis of tumor and calculating the tumor volume as major axis (mm) × [minor axis (mm)]² × 1/2.

Measurement of tumor volume was initiated on Day 14 after the tumor transplantation, and significant tumor proliferation-suppressing effect was found in the LPAR1 antagonist administration group from Day 14 (Figure 8B). Found to have antitumor effect, the LPAR1 antagonist can be promising not only for suppressing the lung metastasis of osteosarcoma but also for achieving a remission.

As demonstrated in Figure 2A, LPAR1 was highly expressed also in glioblastoma (GBM) and glioma (LGG) as well as osteosarcoma and sarcoma. Accordingly, the effects of LPAR1 antagonists to suppress metastasis and cell proliferation are also expected to be exerted for glioma. In view of the fact that glioblastoma is the highest malignancy tumor among gliomas as described above, migration ability with addition of LPA was analyzed with glioblastoma cell strains. Analysis was performed to determine whether the mobility of glioblastoma cells is augmented in the presence of LPA. In a Transwell chamber, the human glioblastoma cells Onda7 (obtained from JCRB Cell Bank), YKG-1 (obtained from JCRB Cell Bank), and 42-MG-BA (obtained from DSMZ) were seeded at 1 × 10⁵ cells each, with addition of 100 nM LPA as a migration factor to the underlying chamber, and 24 hours thereafter cells that migrated to the underlying chamber were stained with crystal violet and quantified. Before being collected, the cells were pretreated with 100 nM Ki16425, which is an LPAR antagonist, for 1 hour, and subjected to analysis of migration ability still in the presence of 100 nM Ki16425. While migration ability was largely enhanced by LPA in all the cell types, cells pretreated with Ki16425 exhibited largely reduced migration ability even when LPA was added (Figure 9).

Next, examination was carried out to determine whether LPAR1 antagonists had effects on viabilities of glioblastoma cells. On a 96-well plate, 1500 to 3000 cells of each of the human glioblastoma cells Onda7, YKG-1, and 42-MG-BA were seeded and treated with any of the LPAR1 antagonists Ki16425, BMS-986020, and ONO-7300243 in different concentrations, and the cell viabilities 72 hours thereafter were determined with CellTiter-Gro reagent (Figure 10A). Reduced cell viabilities were found for all the cell types and all the LPAR1 antagonists.

Next, viabilities of glioblastoma cells with LPAR1 knockdown were analyzed (Figure 10B). Onda7 and YKG-1 cells were seeded and then treated with any of two types of siRNA targeting LPAR1 (Dharmacon), and cell viabilities 4 days thereafter were determined with CellTiter-Gro reagent. Significantly reduced viabilities were found in all the cell types when LPAR1 expression was suppressed with siRNA. The finding of increased levels of cleaved PARP (Cl-PARP), which is an apoptosis marker, suggested that apoptosis was induced through LPAR1 knockdown. From these results, reducing LPAR1 expression can be expected to cause metastasis-suppressing and cell proliferation-suppressing effects even in glioblastoma. That is, LPAR1 antagonists are expected to be applicable as molecular target drugs in treatment.

Examination was carried out to determine whether an LPAR1 antagonist exerts antitumor effect even for glioblastoma. Into each female SCID-beige mouse, 2 × 10⁵ Onda7 cells with Akaluc luciferase introduced therein were intracranially transplanted. Three weeks after the transplantation, oral administration according to a dosing schedule of continuous administration of the LPAR1 antagonist BMS-986020 of 50 mg/kg or 100 mg/kg for 5 consecutive days and withdrawal for 2 days was initiated (Figure 11A). For tumor volume, the enzymatic activity of AkaLuc luciferase stably expressed in Onda7 cells was quantified with an IVIS imaging system (Figure 11B).

The BMS-986020 administration group was found to have the proliferation of glioblastoma cells suppressed and exhibited relative tumor volume almost comparable to that at the transplantation from 2 weeks after the initiation of administration (5 weeks after the transplantation) even for the 50 mg/kg administration, and significant difference was found from 7 weeks after the transplantation as compared with a control. The LPAR1 antagonist was confirmed to exhibit antitumor effect even for glioblastoma.

As demonstrated hereinbefore, the present inventors revealed that LPAR1 is largely involved in the metastasis mechanism of osteosarcoma. On the basis of the clarified metastasis mechanism, the present inventors further demonstrated with a mouse model that the metastasis can be suppressed by administering an LPAR1 antagonist. As described above, osteosarcoma involves lung metastasis with high rate, no effective therapeutic method is available for osteosarcoma patients who have undergone lung metastasis, and most of the causes of patient death are respiratory failure due to lung metastasis. In light of these facts, the consequence that the lung metastasis of osteosarcoma can be suppressed with an LPAR1 antagonist is very useful for providing a novel therapeutic method for osteosarcoma to achieve improved prognosis. In addition, the present inventors have demonstrated that the proliferation of osteosarcoma cells is suppressed by suppressing the expression of LPAR1 and apoptosis is induced. That is, not only metastasis of but also the proliferation of osteosarcoma is suppressed by administering an LPAR1 antagonist. Treatment of osteosarcoma by suppressing the LPA-LPAR1 interaction is expected to be a novel therapeutic method capable of suppressing the metastasis and proliferation of osteosarcoma.

It was further demonstrated that LPAR1 antagonists have effects not only for osteosarcoma but also for glioma, in which LPAR1 is highly expressed as well. Depending on the site where it occurs, gliomas is intractable tumor that is difficult to treat surgically and difficult to remove tumor cells completely. LPAR1 antagonists are also expected to be a novel treatment option for glioma.

## Claims

1. A pharmaceutical composition for treating osteosarcoma or glioma, the pharmaceutical composition containing a compound that suppresses LPAR1 expression as an active ingredient.

2. The pharmaceutical composition for treating osteosarcoma or glioma according to claim 1, wherein the compound that suppresses LPAR1 expression is an LPAR1 antagonist or a nucleic acid.

3. The pharmaceutical composition for treating osteosarcoma or glioma according to claim 2, wherein the LPAR1 antagonist is a low-molecular-weight compound.

4. The pharmaceutical composition for treating osteosarcoma or glioma according to claim 3, wherein the LPAR1 antagonist is any of ONO-7300243, BMS-986020, Ki16425, ONO-3080573, ONO-9780307, ONO-9910539, Ki16198, AM095, AM966, SAR100842, BMS-986278, and analogs thereof.

5. A method of assisting prognosis for an osteosarcoma patient, wherein LPAR1 expression in disease tissue obtained from the patient is analyzed, and if the LPAR1 expression is high, the risk of metastasis is predicted to be high.

6. A drug selection method, wherein LPAR1 expression in disease tissue is analyzed, and if LPAR1 expression is detected, the disease is determined to be a subject for LPAR1 antagonist administered.

7. A screening method for a pharmaceutical composition for treating osteosarcoma or glioma, the screening method including the steps of: bringing a candidate substance into contact with a cell with appreciable LPAR1 expression; and measuring the change of LPAR1 expression.
